# EUROPEAN PATENT SPECIFICATION

(11) **EP 4 464 299 B1**
(45) Date of publication and mention of the grant of the patent: **26.11.2025**
(21) Application number: 23174204.0
(22) Date of filing: 19.05.2023
(51) Int. Cl.: A61J 1/20, A61B 6/10, A61M 5/178

(54) **FLIPPING STATION FOR TRANSFERRING FLUIDS**
WENDESTATION ZUM UMLEITEN VON FLÜSSIGKEITEN
STATION DE RETOURNEMENT POUR TRANSFÉRER DES FLUIDES

(43) Date of publication of application: 20.11.2024
(73) Proprietor: Trasis S.A., 4430 Ans (BE)
(72) Inventor: MASSET, Julien, Eghezee (BE); WARNIER, Corentin, Trooz (BE); GÜLSU, Ugur, Fleron (BE); VRIAMONT, Charles, Hannut (BE); BAPLUE, Frédéric, Tinlot (BE); MORELLE, Jean-Luc, Liege (BE)
(74) Representative: AWA Benelux

(56) References cited:
- US-A1- 2010 049 144
- US-B1- 7 425 208

## Description

### Field of the Invention

The present invention relates to a device for transferring fluids between a container containing a liquid, for example a radioactive substance solution, and a dedicated apparatus for its preparation. The invention relates more particularly to a piercing needle holding device.

### Background and Prior Art

### Nuclear medicine

Cancer is considered one of the biggest health problems in the world today. According to recent reports, more than 10,000 hospitals worldwide use radionuclides for in vivo diagnosis or therapy, and about 35 million patients receive cancer therapy with radionuclides each year *(*European Commission: supply of medical radioisotopes. Available online: https://euratom-supply.ec.europa.eu/activities/supply-medical-radioisotopes_en, accessed on March 24th, 2023*).* In addition to cancer therapy, radionuclides are used in nuclear medicine for the diagnosis and treatment of various diseases, such as cardiovascular and brain diseases.

Different radionuclides are used in different areas of nuclear medicine. Taking into account the half-life and decay emission of the respective radionuclides, they are used for imaging by positron emission tomography (PET) or single-photon emission computed tomography (SPECT) and for therapy by means of a, b⁻, conversion and/or Auger electron emission. Beta particles, alpha particles and Auger electrons can irradiate tissue volumes with multicellular, cellular and subcellular dimensions thanks to the different linear energy transfer (LET), defined as the amount of energy transferred from a travelling particle per unit length to the surrounding material *(*A. Ku et al., Auger electrons for cancer therapy-a review. EJNMMI Radiopharma. Chem. 2019, 4, 1-36*).*

### Radiometals radiochemistry

Radiometals have been extensively used in clinical diagnostics over the last three decades, particularly, ⁹⁹mTc, ⁶⁷Ga and ¹¹¹In for SPECT and, more recently, ⁶⁸Ga, ⁸⁹Zr and ⁶⁴Cu for PET. On the other hand, therapeutic radiometals in clinical use are nowadays mainly limited to ¹⁷⁷Lu, ⁹⁰Y, ⁸⁹Sr, ²²³Ra, ²²⁵Ac, ²¹¹At, ²¹³Bi, ⁴⁷Sc, ¹⁶¹Tb and ²²⁷Th (See: H. Ahmadzadehfar et al., Therapeutic response and side effects of repeated radioligand therapy with 177Lu-PSMA-DKFZ-617 of castrate-resistant metastatic prostate cancer. Oncotarget 2016, 7, 12477-12488*;* A.K. Pfeifer et al., Peptide Receptor Radionuclide Therapy with Y-DOTATOC and 177Lu-DOTATOC in Advanced Neuroendocrine Tumors: Results from a Danish Cohort Treated in Switzerland. Neuroendocrinology 2011, 93, 189-196; K. Yamada et al., Concurrent use of Sr-89 chloride with zoledronic acid is safe and effective for breast cancer patients with painful bone metastases. Exp. Ther. Med. 2012, 3, 226-230*;* R.B. Den et al., Ra-223 treatment for bone metastases in castrate-resistant prostate cancer: Practical management issues for patient selection. Am. J. Clin. Oncol. Cancer Clin. Trials 2019, 42, 399-406*;* B. Nelson et al., Targeted Alpha Therapy: Progress in Radionuclide Production, Radiochemistry, and Applications. Pharmaceutics 2021, 13, 49-75*;* E. Hindie et al., Dose Deposits from 90Y, 177Lu, 111In, and 161Tb in Micrometastases of Various Sizes: Implications for Radiopharmaceutical Therapy. J. Nucl. Med. 2016, 57, 759-764; K.A. Domnanich et al. 47Sc as useful b- emitter for the radiotheragnostic paradigm: A comparative study of feasible production routes. EJNMMI Radiopharm. Chem. 2017, 2, 1-17*).* ²²³Ra is the only approved radionuclide by health authorities for targeted alpha therapy to extend survival.

Radiometals are mainly produced by cyclotron or nuclears reactors (P. Daya, Increasing Radiopharmaceutical Production with Cyclotrons. News from the International Atomic energy Agency (mai 2011). Accessible online: https://www.iaea.org/newscenter/news/increasinq-radiopharmaceutical-production-with-cyclotrons, accessed on March 24th, 2023), shipped and supplied as a liquid acidic solution (pH 1-2).

### Automation of the Labeling Procedure Towards GMP Production

Once produced, a radiometal need to be labelled to a vector molecule to form a radiopharmaceutical that would eventually target the disease. Radiopharmaceutical products for clinical applications must follow the universal requirements for the drug products. Drug products must for the sake of patient safety be of good quality. The European Pharmacopoeia (EP) and likewise the United States Pharmacopoeia (USP) and the Japanese Pharmacopoeia (JP) set the minimum quality standards for drug products in general and also for radiopharmaceuticals. Recently the European Directorate for the Quality of Medicines & HealthCare (EDQM) published the "Guide for the elaboration of monographs on Radiopharmaceutical Preparations" (European Pharmacopoeia 10.0. Radiopharmaceutical Preparations; European Directorate for the Quality of Medicines & HealthCare: Strasbourg, France, 2016; pp. 884-887), which is a useful resource for setting quality parameters and specifications for new radiopharmaceuticals.

When planning for the first-in-man clinical trial, the quality of labeling solution, as well as overall product quality, should be assessed against EP quality standards to guarantee a good quality product that reproducibly meets all the predefined specifications. When evolving towards later stages of clinical studies and especially towards commercialization of a drug product, full Good Manufacturing Practice (GMP) will apply as published by the European Commission's EudraLex volume 4 of "The rules governing medicinal products in the European Union" (Pharmaceutical Legistration, EudraLex Volume 4, Good manufacturing practice (GMP) Guidelines. Available online: http://www.it-asso.com/qxp/eudralex_v27/contents/homev4.htm, accessed on March 24th, 2023*).*

Automated modules are regularly used to ensure consistency of labeling the radionuclide to the target for producing the radiopharmaceutical under GMP requirements. In addition, an automated system can minimize human intervention and guarantee better control of sterility and pyrogenicity of radiopharmaceuticals *(*P. Elsinga et al., Guidance on current good radiopharmacy practice (cGRPP) for the small-scale preparation of radiopharmaceuticals. Eur. J. Nucl. Med. Mol. Imaging 2010, 37, 1049-1062*). A* current trend in the development of automated systems is moving from the fixed tubing system, that have characterized a large variety of synthesis modules over the years, to a "disposable sterile cassette"-based system (S. Boschi et al., Automation synthesis modules review. Appl. Radiat. Isot. 2013, 76, 38-45). The disposable cassette systems are intended for single-use and are designed for one or more specific syntheses. For example, ⁶⁶Ga/¹⁷⁷Lu/⁹⁰Y/¹¹¹In DOTA-peptides have been synthesized with high purity, high radiochemical yield and acceptable synthesis time using such a system (M. Petrik et al., Radiolabelling of peptides for PET, SPECT and therapeutic applications using a fully automated disposable cassette system. Nucl. Med. Commun. 2011, 32, 887-895).

The development of an automated procedure for therapeutic radiolabeling with novel radionuclides is a considerable challenge and several factors have to be taken into consideration. Firstly, the final product has to be purified from possible free activity, i.e. radionuclide that is not associated with the conjugate molecule, which is performed using cartridge-based methods. Furthermore, to achieve high labeling efficiencies of conjugates, the concentrations of the reagents used must be high and, therefore, low volumes are used. These small volumes of reagents require transfer through the system without loss or with minimal loss of solution. It is also important to prevent contamination of the product with metallic impurities, which may compete with the radionuclide for the limited number of chelating sites on the conjugate molecule *(*M. Asti et al. Influence of cations on the complexation yield of DOTATATE with yttrium and lutetium: A perspective study for enhancing the 90Y and Lu labeling conditions. Nucl. Med. Biol. 2012, 39, 509-517*;* Z. Talip et al., A Step-by-Step Guide for the Novel RadiometalProduction for Medical Applications: Case Studies with 68Ga, 44Sc, 177Lu and 161Tb. Molecules 2020, 25, 966-995). This implies that metallic transfer systems, such as needles, ought to be avoided where transferring the acidic radionuclide solutions (pH 1-2).

Apart from the pharmaceutical regulations, it is of key importance to also consult the national radioprotection legislation. Radiation to the operator must be minimized, from the transfer of the activity to the reception of the radiopharmaceutical finished product. To this purpose, automated radiosynthesizers are designed for use in an appropriate hot cell that provides suitable radiation shielding to protect the operator from potential radiation dose during the radiolabeling process. However, operator radioprotection during the cassette and consumable set up is usually not well-assured as the operator must prick manually with a needle connected to the cassette trough the septum of the crimp capped vial containing the radionuclide activity.

### Problem to be solved

Metallic radionuclides, radiometals, have an important role in nuclear medicine. Their straightforward coordination radiochemistry allows for a large variety of applications. Radiometals are supplied as a liquid acidic solution (pH 1-2) of very low volume (~0.5-4 mL) in crimp capped vials. The shape and size of the vials depend on the supplier. For radioprotection purposes, vials are inserted in armored containers. Supply activity can be as high as 150 GBq, possibly exposing the operator to high amount of radiation. On the other hand, radiometal radiochemistry is very sensitive to metallic impurities. To perform the transfer of the activity, the operator manually plunges a metallic needle into the vial containing the radionuclide. This practice does not protect the operator from radiation and may result in metallic leachable from the needle within the radionuclide solution, harming the subsequent radiolabeling. Furthermore, depending of the vial shape, a significant quantity the starting activity remains in the vial after aspiration of the radionuclide solution. The present invention is intended to provide a device that allows a quantitative transfer of the activity to the cassette in a safe way for the operator, using metal-free tools and adaptable to all sizes and shapes of radionuclide-containing vials commercially available.

Document US 7,425,208 B1 discloses a needle assembly structured to facilitate substantially complete removal, if not the complete removal, of a pharmaceutical or other composition from a vial or like container. The needle assembly comprises a delivery needle having an elongated first portion of sufficient length to extend within and along the entire length of the interior of the vial. A distal end of the delivery needle is disposable in confronting engagement with an interior floor portion of the vial. The delivery needle is connected to a base and is concurrently movable relative thereto while maintaining the distal end of the first portion in confronting relation to the vial floor. Complete removal of the pharmaceutical composition from the vial is thereby facilitated.

### Aims of the Invention

The present invention aims the quantitative transfer of a liquid from a crimp capped vial to another vial or a cassette.

A further goal of the present invention is to proceed to the liquid transfer using metal-free tools.

A further aim of the present invention is to adapt to all sizes and shapes of small-volume crimp capped vials commercially available.

In particular, it is aimed to quantitatively and precisely transfer a radioactive substance containing liquid in a safe way for the operator.

### Summary of the Invention

The invention is disclosed in the claims. A first aspect of the present invention relates to a device for performing by flipping-over a quantitative liquid transfer from a crimp capped vial to another vial, container or a cassette, said device comprising an extension tube equipped with a spike on one end, a shielded pot with a lid, intended to enclose the crimp capped vial, and a component made of:
- a lower cylindrical part with a beveled cylindrical notch, able to receive in tight insertion a crimp capped vial, said notch having an aperture on a side of the lower cylindrical part;
- an upper cylindrical part in communication with the lower cylindrical part and comprising a hollow cylindrical protrusion for guiding the extension tube equipped with a spike, a groove being provided in said upper cylindrical part for keeping the extension tube in place during the handling of said device;
- a central cylindrical part, provided between the lower and the upper cylindrical parts, comprising a furrow to ensure a correct positioning of the extension tube during the transfer of the fluid to another vial, container or a cassette, a round rail being provided on each side of the central cylindrical part allowing the shielded pot and its lid to be respectively anchored on said component;
said aperture being extended in the respective central and upper cylindrical parts and said spike being connected to a luer lock ring.

According preferred embodiment, the device further comprises one or more of the following characteristics :
- the upper cylindrical part with a beveled cylindrical notch is designed to be adaptable to a variety of sizes and shapes of small volume commercially available crimp capped vials;
- the spike is made of a metal-free material;
- said component is made of a rigid plastic material.

Another aspect of the present invention relates to a method of quantitatively transferring a liquid from a crimp capped vial to another vial, container or a cassette using the device according to anyone of the preceding claims, said method comprising the successive steps of:
- inserting the spike connected to a luer lock ring in the aperture of the beveled notch of the first cylindrical part, maintaining the spike downwards so that the luer lock ring is stopped by an upper flange of the beveled notch, while the extension tube is inserted in the extended aperture in the respective central and upper cylindrical parts;
- removing the lid of a shielding pot enclosing the vial which contains the liquid to be transferred and placing said lid onto the upper round rail of the central cylindrical part of said element;
- positioning the assembly of said element and said lid on top of the lead pot, thanks to the lower round rail, and pressing the same down until a septum of the vial located inside the lead pot is pierced by the spike;
- connecting the extension tube on its end opposite to the spike to the other vial, container or to a cassette;
- flipping over the element and proceeding to the quantitative liquid transfer.

Preferably, the liquid to be transferred contains a radioactive substance, still preferably a radiometal, the radiometal being still preferably selected from the group consisting of 99mTc, 67Ga, 111In, 68Ga, 89Zr, 64Cu, 67Cu, 177Lu, 90Y, 89Sr, 223Ra, 225Ac, 211At, 213Bi, 47Sc, 161Tb and 227Th.

Advantageously the liquid transfer is operated till the remaining activity in the original vial (12) is less than 3%.

### Brief Description of the Drawings

FIG. 1 represents different views of an embodiment of the device according to the present invention.
FIG. 2A is an exploded view of the device of Fig. 1, in use for the transfer of a radioactive substance containing liquid.
FIG. 2B represents two views of an embodiment of the device according to the present invention, illustrating the placement of the spike set inside said device.
FIG. 2C is a cross-sectional view of an embodiment of the device according to the present invention in use, representing the placement of the device for the transfer of a radioactive substance containing liquid before the flipping over step.
FIG. 2D is a cross-sectional view of an embodiment the device according to the present invention, in the position ready for the transfer a radioactive substance containing liquid.

### Description of a Preferred Embodiments of the Invention

The present invention relates to a device allowing a quantitative transfer of a liquid contained in a vial to a cassette or another vial or container, without the use of a metal needle. The device shall be designed to be adaptable to different sizes and shapes of vials that can be easily found off the shelf and shall protect the operator from radiation when the liquid to be transferred contains a radioactive substance.

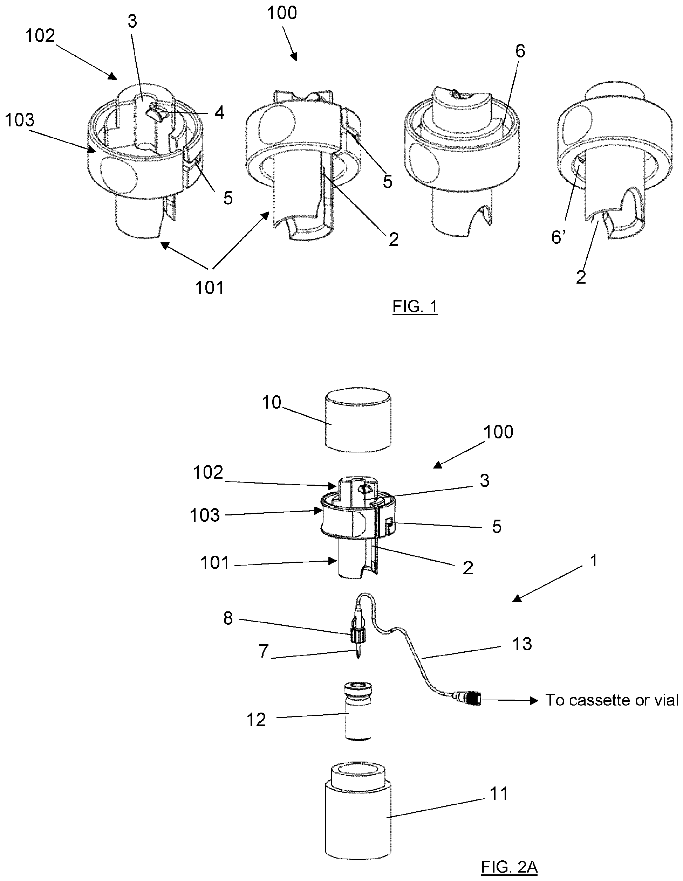

As illustrated by FIG. 1 and FIG. 2A-2B, the device component 100 according to the present invention comprises a lower cylindrical part 101 with a beveled cylindrical notch in which a crimped vial 12 containing a liquid can be tightly inserted. This lower cylindrical part 101 with its beveled cylindrical notch fits most standard small volume crimp capped vials 12 and has an aperture 2 on one side.

The device component 100 is further provided with an upper cylindrical part 102 connected to the first cylindrical part 101. This upper cylindrical part 102 comprises a hollow cylindrical protrusion 3 to guide in use an extension tube 13 equipped with a spike 7 towards the cap of the vial 12 containing the liquid to be transferred. A groove 4 is provided in the upper cylindrical part 102 for keeping the extension tube 13 in place during the manipulation.

In the center part of the device component 100, a central cylindrical part 103 is provided, comprising a furrow 5 ensuring a correct positioning of the extension tube 13 during the transfer of the fluid to the other container or cassette. The central part 103, which is preferably wider than the respective lower and upper cylindrical parts 101, 102, has a round rail on each side 6, 6' that allow a shielded pot 11 and its lid 10 to be anchored in use, in the case of transferring a liquid containing a radioactive substance.

It is to be noted here that the terms "lower" and "upper" terms refer to parts of element 100 in its normal vertical orientation when inserting the spike 7 into the septum of the vial 12. For the liquid transfer step, the element 100 connected to the shielding pot 11 and lid 10 will be flipped over, that means put upside down.

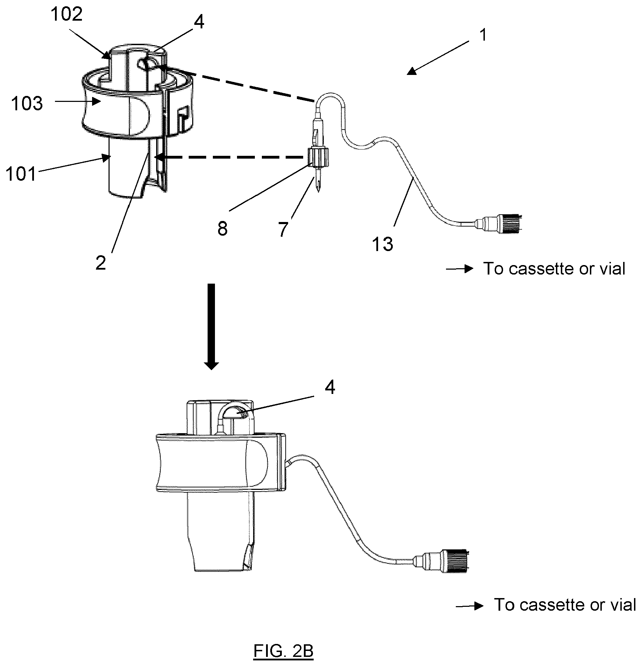

Operation of the device 1 to transfer a liquid containing a radioactive substance confined in a crimp capped vial 12 is illustrated in FIG. 2A-2D. Firstly, another vial or a cassette is connected to the end of the extension tube 13 opposite to the end carrying the spike 7, the spike being connected on this other end to a luer lock ring 8. This assembly of spike 7 and luer lock ring 8 will be referred to hereafter as a spike set 9. The spike set 9 is further inserted into the aperture 2 of the beveled notch of the lower cylindrical part 101, maintaining the spike 7 downwards and so that the luer lock ring 8 is stopped by an upper flange of the notch (see FIG. 2B).

To this end the aperture 2 of the beveled notch of the lower cylindrical part 101 is extended by an aperture in the central part 103 and in the upper cylindrical part 102, so that the spike set 9 and the extension tube 13 can be both inserted in the component laterally, while the spike set 9 itself is, as mentioned, inserted in the beveled notch aperture 2 below the central part 103 (see FIG. 2B).

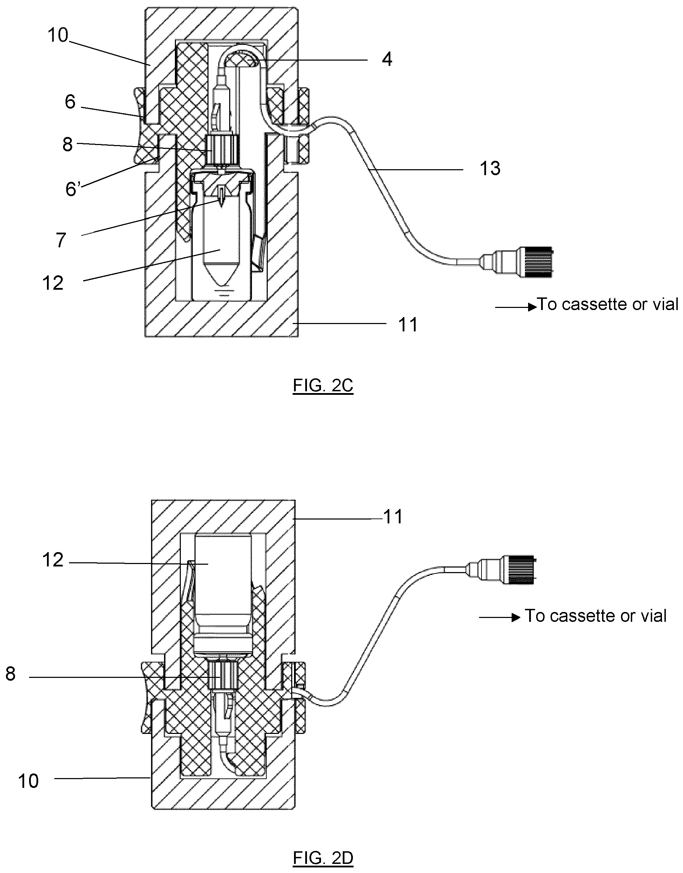

The lid 10 of a shielding pot 11, usually a lead pot, enclosing the vial 12 which contains the radionuclide solution is removed and placed onto an upper round rail 6 of the component 100. The component-lid assembly is further positioned on top of the lead pot 11 and pressed down until the septum of the vial 12 located inside the lead pot 11 is pierced by the spike 7 in the component 100 (FIG. 2C). As the pressure is applied on the shielded lid 10 of the lead pot 11, the operator is protected from radiation.

The whole is then manually flipped over, so that the vial 12 is upside down on the spike 7 (FIG. 2D). The fluid transfer is made either via aspiration by the synthesizer towards a cassette, or by depression to another vial. Since the vial 12 is upside down on the spike 7, the transfer is quantitative. Compared to the state of the art method, use of the present device 1 reduces the remaining activity in the supplier vial and its variability, permits an easier and metal-free connection to the cassette which guarantees high labeling yields, and assures an enhanced radioprotection of the operator.

### EXAMPLES

| # | Method | Vial shape | Vial volume (mL) | Sample volume (µL) | Isotope | Starting activity (MBq) | Residual activity after withdrawing (%) |
|---|---|---|---|---|---|---|---|
| 1 | Withdrawing sample with a needle (state of the art) | V-Vial | 2 | 75 | 177Lu | 132,5 | 18,3 |
| 2 | Withdrawing sample with a needle (state of the art) | Flat Bottom | 10 | 150 | 177Lu | 212,1 | 20,1 |
| 3 | Present invention | Flat bottom | 10 | 2000 | 18F | 253,2 | 0,9 |
| 4 | Present invention | V-Vial | 2 | 75 | 18F | 120,2 | 1,7 |
| 5 | Present invention | V-Vial | 2 | 75 | 177Lu | 145,5 | 2,2 |
| 6 | Present invention | V-Vial | 2 | 75 | 177Lu | 144,1 | 1,9 |
| 7 | Present invention | V-Vial | 2 | 75 | 177Lu | 139,5 | 1,8 |
| 8 | Present invention | Flat bottom | 10 | 150 | 177Lu | 186,3 | 2,7 |
| 9 | Present invention | Flat bottom | 10 | 150 | 177Lu | 187,0 | 1,6 |
| 10 | Present invention | Flat bottom | 10 | 150 | 177Lu | 191,5 | 1,9 |

Experiments 3-10 demonstrate that the liquid transfer is almost quantitative exhibiting an activity recovery far better than with the state of the art method (experiments 1-2). Whatever the isotope, shape and size of the vial, a recovery > 97% is achieved with the device and method according to the present invention.

### List of reference symbols

- 1: Device for a quantitative liquid transfer from a crimp capped vial to another vial, container or a cassette
- 100: Component
- 101: Lower cylindrical part with beveled cylindrical notch
- 102: Upper cylindrical part
- 103: Central cylindrical part
- 2: Aperture in the cylindrical notch of the lower cylindrical part
- 3: Hollow cylindrical protrusion of the upper cylindrical part
- 4: Groove
- 5: Furrow
- 6, 6': Round rail on each side of the central cylindrical part
- 7: Spike
- 8: Luer lock ring
- 9: Spike set
- 10: Lid
- 11: Lead pot
- 12: Vial containing the fluid to be transferred
- 13: Extension tube

## Claims

1. A device (1) for performing by flipping-over a quantitative liquid transfer from a crimp capped vial (12) to another vial, container or a cassette, said device (1) comprising an extension tube (13) equipped with a spike (7) on one end, a shielded pot (11) with a lid (10), intended to enclose the crimp capped vial (12) and a component (100) made of:
- a lower cylindrical part (101) with a beveled cylindrical notch, able to receive in tight insertion the crimp capped vial (12), said notch having an aperture (2) on a side of the lower cylindrical part (101);
- an upper cylindrical part (102) in communication with the lower cylindrical part (101) and comprising a hollow cylindrical protrusion (3) for guiding the extension tube (13) equipped with a spike (7), a groove (4) being provided in said upper cylindrical part (102) for keeping the extension tube (13) in place during the handling of said device(1);
- a central cylindrical part (103), provided between the lower and the upper cylindrical parts (101, 102), comprising a furrow (5) to ensure a correct positioning of the extension tube (13) during the transfer of the fluid to another vial, container or a cassette, a round rail (6, 6') being provided on each side of the central cylindrical part (103) allowing the shielded pot (11) and its lid (10) to be respectively anchored on said component (100);
said aperture (2) being extended in the respective central and upper cylindrical parts (103, 102) and said spike (7) being connected to a luer lock ring (8)..

2. The device according to claim 1 wherein the upper cylindrical part (101) with a beveled cylindrical notch is designed to be adaptable to a variety of sizes and shapes of small volume commercially available crimp capped vials (12).

3. The device according to claim 1, wherein the spike (7) is made of a metal-free material.

4. The device according to claim 1, wherein said component (100) is made of a rigid plastic material.

5. A method of quantitatively transferring a liquid from a crimp capped vial (12) to another vial, container or a cassette using the device (1) according to anyone of the preceding claims, said method comprising the successive steps of:
- inserting the spike (7) connected to a luer lock ring (8) in the aperture (2) of the beveled notch of the first cylindrical part (101), maintaining the spike (7) downwards so that the luer lock ring (8) is stopped by an upper flange of the beveled notch, while the extension tube (13) is inserted in the extended aperture in the respective central and upper cylindrical parts (103, 102);
- removing the lid (10) of a shielding pot (11) enclosing the vial (12) which contains the liquid to be transferred and placing said lid (10) onto the upper round rail (6) of the central cylindrical part (103) of said element (100);
- positioning the assembly of said element (100) and said lid (10) on top of the lead pot (11), thanks to the lower round rail (6'), and pressing the same down until a septum of the vial (12) located inside the lead pot (11) is pierced by the spike (7);
- connecting the extension tube (13) on its end opposite to the spike (7) to the other vial, container or to a cassette;
- flipping over the element (100) and proceeding to the quantitative liquid transfer.

6. The method according to claim 5, wherein the liquid to be transferred contains a radioactive substance.

7. The method according to claim 6, wherein the radioactive substance is a radiometal.

8. The method according to claim 7, wherein the radiometal is selected from the group consisting of 99mTc, 67Ga, 111In, 68Ga, 89Zr, 64Cu, 67Cu, 177Lu, 90Y, 89Sr, 223Ra, 225Ac, 211At, 213Bi, 47Sc, 161Tb and 227Th.

9. The method according to claim 5, wherein the liquid transfer is operated till the remaining activity in the original vial (12) is less than 3%.

## Patentansprüche

1. Vorrichtung (1) zum Durchführen einer Wendung einer quantitativen Flüssigkeitsumleitung von einem Fläschchen mit Bördelkappe (12) zu einem anderen Fläschchen, Behälter oder einer Kassette, wobei die Vorrichtung (1) ein Verlängerungsrohr (13) umfasst, das mit einem Dorn (7) an einem Ende, einem abgeschirmten Gefäß (11) mit einem Deckel (10) der ausgelegt ist, um das Fläschchen mit Bördelkappe (12) zu umschließen, und einer Komponente (100) ausgestattet ist, hergestellt aus:
- einem unteren zylindrischen Teil (101) mit einer abgeschrägten zylindrischen Einkerbung, die dazu geeignet ist, in fester Einführung das Fläschchen mit Bördelkappe (12) aufzunehmen, wobei die Einkerbung eine Öffnung (2) auf einer Seite des unteren zylindrischen Teils (101) aufweist;
- einem oberen zylindrischen Teil (102) in Kommunikation mit dem unteren zylindrischen Teil (101) und umfassend einen hohlen zylindrischen Vorsprung (3), zum Führen des Verlängerungsrohrs (13), das mit einem Dorn (7) ausgestattet ist, wobei eine Rille (4) in dem oberen zylindrischen Teil (102) bereitgestellt ist, um das Verlängerungsrohr (13) während der Handhabung der Vorrichtung (1) in seiner Position zu halten;
- einem zentralen zylindrischen Teil (103), der zwischen dem unteren und dem oberen zylindrischen Teil (101, 102) bereitgestellt ist, umfassend eine Furche (5), um eine korrekte Positionierung des Verlängerungsrohrs (13) während der Umleitung der Flüssigkeit an ein anderes Fläschchen, einen Behälter oder eine Kassette sicherzustellen, wobei eine runde Schiene (6, 6') auf jeder Seite des zentralen zylindrischen Teils (103) bereitgestellt ist, die ermöglicht, dass das abgeschirmte Gefäß (11) und sein Deckel (10) jeweils auf der Komponente (100) verankert sind,
wobei die Öffnung (2) in den entsprechenden zentralen und oberen zylindrischen Teil (103, 102) verlängert ist und der Dorn (7) mit einem Luer-Verschlussring (8) verbunden ist.

2. Vorrichtung nach Anspruch 1, wobei der obere zylindrische Teil (101) mit einer abgeschrägten zylindrischen Einkerbung entworfen ist, um an eine Vielzahl von Größen und Formen an kommerziell erhältlicher Fläschchen mit Bördelkappe mit kleinem Volumen (12) angepasst werden zu können.

3. Vorrichtung nach Anspruch 1, wobei der Dorn (7) aus einem metallfreiem Material hergestellt ist.

4. Vorrichtung nach Anspruch 1, wobei die Komponente (100) aus einem starren Kunststoffmaterial hergestellt ist.

5. Verfahren zum quantitativen Umleiten einer Flüssigkeit von einem Fläschchen mit Bördelkappe (12) zu einem anderen Fläschchen, Behälter oder einer Kassette unter Verwendung der Vorrichtung (1) nach einem der vorhergehen Ansprüche, wobei das Verfahren die folgenden sukzessiven Schritte umfasst:
- Einführen des Dorns (7), der mit einem Luer-Verschlussring (8) verbunden ist, in die Öffnung (2) der abgeschrägten Einkerbung des ersten zylindrischen Teils (101), Halten des Dorns (7) nach unten, so dass der Luer-Verriegelungsring (8) durch einen oberen Flansch der abgeschrägten Einkerbung gestoppt wird, während das Verlängerungsrohr (13) in die verlängerte Öffnung in dem entsprechenden zentralen und oberen zylindrischen Teil (103, 102) eingeführt wird,
- Entfernen des Deckels (10) eines abgeschirmten Gefäßes (11), das das Fläschchen (12) umschließt, das die Flüssigkeit enthält, die umgeleitet werden soll, und Platzieren des Deckels (10) auf die obere runde Schiene (6) des zentralen zylindrischen Teils (103) des Elements (100);
- Positionieren der Einheit des Elements (100) und des Deckels (10) auf die obere Seite des Blutgefäßes (11) dank der unteren runden Schiene (6') und Drücken derselben nach unten, bis das Septum des Fläschchens (12), das sich innerhalb des Bleigefäßes (11) befindet, durch den Dorn (7) durchstochen ist;
- Verbinden des Verlängerungsrohrs (13) auf seinem Ende gegenüber dem Dorn (7) mit dem anderen Fläschchen, Behälter oder einer Kassette;
- Wenden des Elements (100) und Fortfahren mit der quantitativen Umleitung von Flüssigkeit.

6. Verfahren nach Anspruch 5, wobei die Flüssigkeit, die umgeleitet werden soll, eine radioaktive Substanz enthält.

7. Verfahren nach Anspruch 6, wobei die radioaktive Substanz ein Radiometall ist.

8. Verfahren nach Anspruch 7, wobei das Radiometall ausgewählt ist aus der Gruppe, bestehend aus 99mTc, 67Ga, 1111n, 68Ga, 89Zr, 64Cu, 67Cu, 177Lu, 90Y, 89Sr, 223Ra, 225Ac, 211At, 213Bi, 47Sc, 161Tb und 227Th.

9. Verfahren nach Anspruch 5, wobei die Umleitung von Flüssigkeit durchgeführt wird, bis die verbleibende Aktivität im originalen Fläschchen (12) weniger als 3 % beträgt.

## Revendications

1. Dispositif (1) pour effectuer par retournement un transfert quantitatif de liquide d'un flacon à capuchon à sertir (12) vers un autre flacon, récipient ou cassette, ledit dispositif (1) comprenant un tube d'extension (13) équipé d'une pointe (7) à une extrémité, un pot blindé (11) avec un couvercle (10) destiné à enfermer le flacon à capuchon à sertir (12), et un composant (100) constitué :
- d'une partie cylindrique inférieure (101) avec une encoche cylindrique biseautée, apte à recevoir en insertion étanche le flacon à capuchon à sertir (12), ladite encoche présentant une ouverture (2) sur un côté de la partie cylindrique inférieure (101) ;
- une partie cylindrique supérieure (102) en communication avec la partie cylindrique inférieure (101) et comprenant une saillie cylindrique creuse (3) pour guider le tube d'extension (13) équipé d'une pointe (7), une rainure (4) étant prévue dans ladite partie cylindrique supérieure (102) pour maintenir le tube d'extension (13) en place pendant la manipulation dudit dispositif (1);
- une partie cylindrique centrale (103), prévue entre les parties cylindriques inférieure et supérieure (101, 102), comprenant un sillon (5) pour assurer un positionnement correct du tube d'extension (13) pendant le transfert du fluide vers un autre flacon, récipient ou cassette, un rail rond (6, 6') étant prévu de chaque côté de la partie cylindrique centrale (103) permettant au pot blindé (11) et à son couvercle (10) d'être respectivement ancrés sur ledit composant (100) ;
ladite ouverture (2) s'étendant dans les parties cylindriques centrale et supérieure respectives (103, 102) et ladite pointe (7) étant reliée à une bague de verrouillage Luer (8).

2. Dispositif selon la revendication 1, **caractérisé en ce que** la partie cylindrique supérieure (101) avec une encoche cylindrique biseautée est conçue pour s'adapter à une variété de tailles et de formes de flacons à capuchon à sertir de petit volume disponibles dans le commerce (12).

3. Dispositif selon la revendication 1, **caractérisé en ce que** la pointe (7) est réalisée en un matériau exempt de métal.

4. Dispositif selon la revendication 1, **caractérisé en ce que** ledit composant (100) est réalisé en une matière plastique rigide.

5. Procédé pour transférer quantitativement un liquide d'un flacon à capuchon à sertir (12) vers un autre flacon, récipient ou cassette à l'aide du dispositif (1) selon l'une quelconque des revendications précédentes, ledit procédé comprenant les étapes successives consistant à :
- insérer la pointe (7) reliée à une bague de verrouillage Luer (8) dans l'ouverture (2) de l'encoche biseautée de la première partie cylindrique (101), maintenir la pointe (7) vers le bas de manière que la bague de verrouillage Luer (8) soit arrêtée par une bride supérieure de l'encoche biseautée, tandis que le tube d'extension (13) est inséré dans l'ouverture prolongée dans les parties cylindriques centrale et supérieure respectives (103, 102) ;
- retirer le couvercle (10) d'un pot de protection (11) renfermant le flacon (12) qui contient le liquide à transférer et placer ledit couvercle (10) sur le rail rond supérieur (6) de la partie cylindrique centrale (103) dudit élément (100) ;
- positionner l'ensemble constitué dudit élément (100) et dudit couvercle (10) sur le pot en plomb (11), grâce au rail rond inférieur (6'), et appuyer sur celui-ci jusqu'à ce qu'un septum du flacon (12) situé à l'intérieur du pot en plomb (11) soit percé par la pointe (7) ;
- raccorder le tube d'extension (13) à son extrémité opposée à la pointe (7) à l'autre flacon, récipient ou cassette ;
- retourner l'élément (100) et procéder au transfert quantitatif du liquide.

6. Procédé selon la revendication 5, **caractérisé en ce que** le liquide à transférer contient une substance radioactive.

7. Procédé selon la revendication 6, **caractérisé en ce que** la substance radioactive est un radiométal.

8. Procédé selon la revendication 7, **caractérisé en ce que** le radiométal est sélectionné parmi le groupe constitué de 99mTc, 67Ga, 111In, 68Ga, 89Zr, 64Cu, 67Cu, 177Lu, 90Y, 89Sr, 223Ra, 225Ac, 211At, 213Bi, 47Sc, 161Tb et 227Th.

9. Procédé selon la revendication 5, **caractérisé en ce que** le transfert de liquide est effectué jusqu'à ce que l'activité restante dans le flacon d'origine (12) soit inférieure à 3 %.
